# FASCICULE DE BREVET EUROPEEN

(11) **EP 1 161 962 B1**
(45) Date de publication et mention de la délivrance du brevet: **02.02.2005**
(21) Numéro de dépôt: 01401446.8
(22) Date de dépôt: 05.06.2001
(51) Int. Cl.: A61M 5/32, A61M 5/50

(54) **Seringue à usage unique comprenant une aiguille rétractable**
Einwegspritze mit einer einziehbaren Nadel
Single use syringe with retractable needle

(30) Priorité: 05.06.2000 FR 0007153
(43) Date de publication de la demande: 12.12.2001
(73) Titulaire: Altair, 14370 Argences (FR)
(72) Inventeur: Arruego, Daniel, 14370 Chicheboville (FR)
(74) Mandataire: Lemoine, Robert

(56) Documents cités:
- WO-A-98/20922
- US-A- 5 120 311
- US-A- 5 263 942
- US-A- 5 300 038
- US-A- 5 728 073

## Description

La présente invention concerne une seringue comprenant un corps tubulaire destiné à recevoir un liquide à injecter ou à prélever, un piston déplaçable axialement dans le corps tubulaire, et une aiguille comportant un conduit interne et portée par un coulisseau situé dans un logement qui est parallèlle et adjacent au corps tabulaire, le logement comportant une extrémité antérieure pourvue d'un alésage conique tandis que le coulisseau est déplaçable axialement dans le logement entre une position extrême rétractée dans laquelle l'aiguille est entièrement contenue dans le logement et une position extrême avancée dans laquelle l'aiguille fait saillie hors du logement, et fait communiquer l'intérieur du corps tubulaire avec le conduit interne de l'aiguille lorsqu'il est dans sa position extrême avancée.

US-A-5 728 073 décrit une seringue de ce type dont le coulisseau ne peut pénêtrer dans l'alésage conique prévu à l'extrémité antérieure du logement prévu pour l'aiguille.

La présente invention a pour objet une seringue du type décrit dans US-A-5 728 073 et qui est caractérisée en ce que les dimensions de l'alésage conique correspondent à celles de l'extrémité antérieure du coulisseau, et en ce que celui-ci, lorsqu'il est dans sa position extrême avancée, est situé dans l'alésage conique et l'obture de manière étanche.

Grâce à cette disposition, le fluide à prélever ou à injecter ne risque pas de fuir lors de l'utilisation de la seringue.

De préférence, le logement comporte une paroi latérale pourvue d'une fente longitudinale se terminant par des élargissements postérieur et antérieur, tandis que le coulisseau comporte des moyens de verrouillage destinés à coopérer avec les élargissements de la fente longitudinale pour l'immobiliser dans ses positions extrêmes.

Lorsque le coulisseau est dans l'une de ses positions extrêmes, les moyens de verrouillage le retiennent dans cette position, évitant qu'il soit déplacé involontairement sous l'action d'une force externe non contrôlée.

Avantageusement, les moyens de verrouillage sont sollicités élastiquement radialement vers l'extérieur et comprennent une embase dont l'étendue correspond à celle des élargissements de la fente longitudinale et qui se prolonge radialement vers l'extérieur par une zone rétrécie dont la largeur correspond à celle de la fente longitudinale puis par un poussoir externe faisant saillie hors de ladite fente.

Etant sollicitée élastiquement vers l'extérieur, l'embase pénètre automatiquement dans l'élargissement de la fente longitudinale qu'elle rencontre lorsque le coulisseau vient dans l'une de ses positions extrêmes. Elle s'oppose donc à ce que le coulisseau puisse être éloigné involontairement de la position extrême dans laquelle il a été placé.

Pour déplacer le coulisseau dans le logement, on conçoit aisément qu'il convient d'exercer une pression sur le poussoir afin de dégager l'embase de l'élargissement dans lequel elle s'étend et d'amener la zone rétrécie devant la fente longitudinale, puis de pousser longitudinalement le poussoir dans le sens voulu.

La seringue conforme à l'invention est en outre caractérisée en ce qu'elle comprend des moyens de sécurité pour immobiliser définitivement le coulisseau dans sa position extrême rétractée lorsque celui-ci est ramené dans cette position après avoir été déplacé vers ou jusqu'à sa position extrême avancée.

Grâce à ses moyens de sécurité, la seringue selon l'invention ne peut plus être réutilisée lorsqu'elle a servi une fois, ou en cas de nécessité, dès que son coulisseau a été éloigné de sa position extrême rétractée d'origine.

Les risques de blessures ou de contamination par piqûre après utilisation de la seringue sont donc éliminés.

De préférence, les moyens de sécurité comprennent un crochet et un verrou portés par le coulisseau, le verrou étant retenu par le crochet lorsque le coulisseau est dans sa position extrême rétractée, une rampe prévue sur la face interne du logement et conformée de façon à déplacer le crochet pour qu'il libère le verrou lors du déplacement du coulisseau de sa position extrême rétractée vers sa position extrême avancée, et un orifice ménagé dans la paroi du logement pour recevoir le verrou libéré lorsque le coulisseau est ramené dans sa position extrême rétractée.

Lorsque le verrou est dans l'orifice, il ne peut en être extrait et immobilise donc définitivement le coulisseau dans sa position extrême rétractée. Une réutilisation de la seringue est par conséquent impossible.

Selon un mode de réalisation particulier, le crochet est prévu à l'une des extrémités d'un premier élément élastique prolongeant le coulisseau axialement vers l'arrière tandis que le verrou est prévu à l'une des extrémités d'un second élément élastique prolongeant également le coulisseau axialement vers l'arrière, les premier et second éléments étant disposés l'un en face de l'autre de telle sorte que leurs extrémités portant respectivement le crochet et le verrou soient adjacentes.

Par ailleurs, les moyens de verrouillage sont prévus à l'autre extrémité du second élément prolongeant le coulisseau axialement vers l'arrière.

Selon le mode de réalisation ci-dessus, le premier élément est de préférence relié au coulisseau par un pont adjacent à son extrémité qui est opposée au crochet tandis que le second élément est relié au coulisseau par un pont approximativement équidistant de ses deux extrémités.

On précisera ici que les deux ponts peuvent être pratiquement diamétralement opposés.

Un mode d'exécution de la présente invention sera décrit ci-après à titre d'exemple nullement limitatif en référence aux dessins annexés dans lesquels :
- la figure 1 est une vue de côté schématique d'une seringue conforme à l'invention, dont le coulisseau est dans sa position extrême rétractée ;
- la figure 2 est une vue en coupe selon la ligne II-II de la figure 1 ;
- la figure 3 est une vue analogue à celle de la figure 1 mais montrant la seringue lorsque le coulisseau est dans sa position extrême avancée ;
- la figure 4 est une vue en coupe selon la ligne IV-IV de la figure 3 ;
- la figure 5 est une vue en coupe analogue à la figure 2 mais montrant le coulisseau après avoir été ramené de sa position extrême avancée dans sa position extrême rétractée ;
- la figure 6 est une vue en perspective schématique de la seringue selon l'invention, dont le coulisseau est dans sa position extrême avancée ;
- la figure 7 est une vue schématique en perspective et en coupe de la seringue dont le coulisseau a été ramené dans sa position extrême rétractée ;
- la figure 8 est une vue en perspective schématique et partielle de l'extrémité postérieure du coulisseau, le crochet ayant libéré le verrou ;
- la figure 9 est une autre vue en perspective schématique et partielle de l'extrémité postérieure du coulisseau, le verrou étant retenu par le crochet ;
- la figure 10 est une vue en coupe schématique selon la ligne X-X de la figure 2 ; et
- la figure 11 est une vue en coupe schématique selon la ligne XI-XI de la figure 4.

La seringue représentée sur les dessins peut être utilisée pour injecter un produit liquide actif biologiquement à un mammifère, y compris l'homme, ou pour prélever un tel produit contenu dans un récipient stérile ou non.

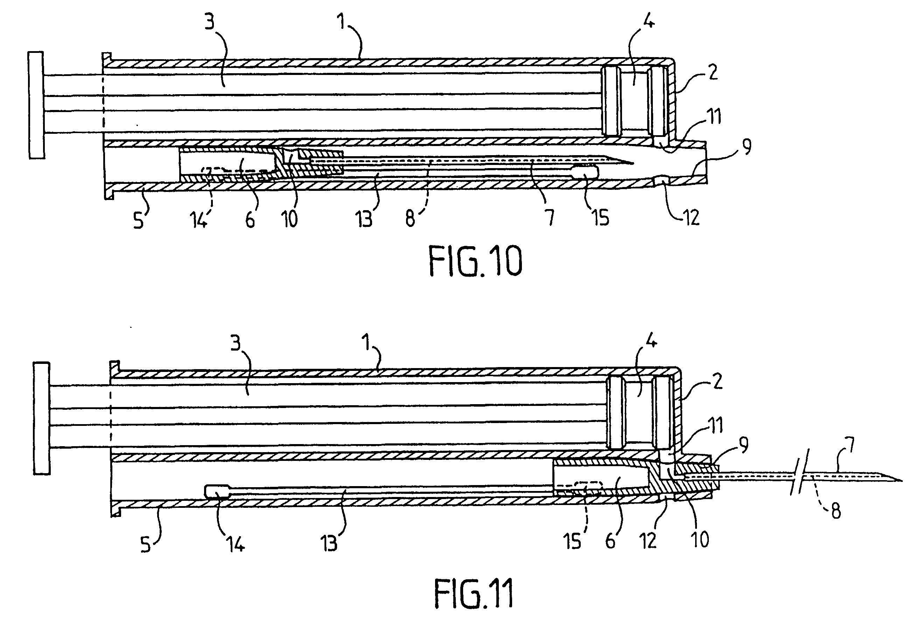

Elle comprend un corps tubulaire 1 dont l'une des extrémités est fermée par un fond 2 et dont l'autre extrémité livre passage à la tige 3 d'un piston mobile 4 pouvant venir contre le fond 2, comme représenté sur les figures 10 et 11.

Conformément à l'invention, la seringue comprend également un logement 5 s'étendant parallèlement au corps tubulaire 1, à l'extérieur de celui-ci.

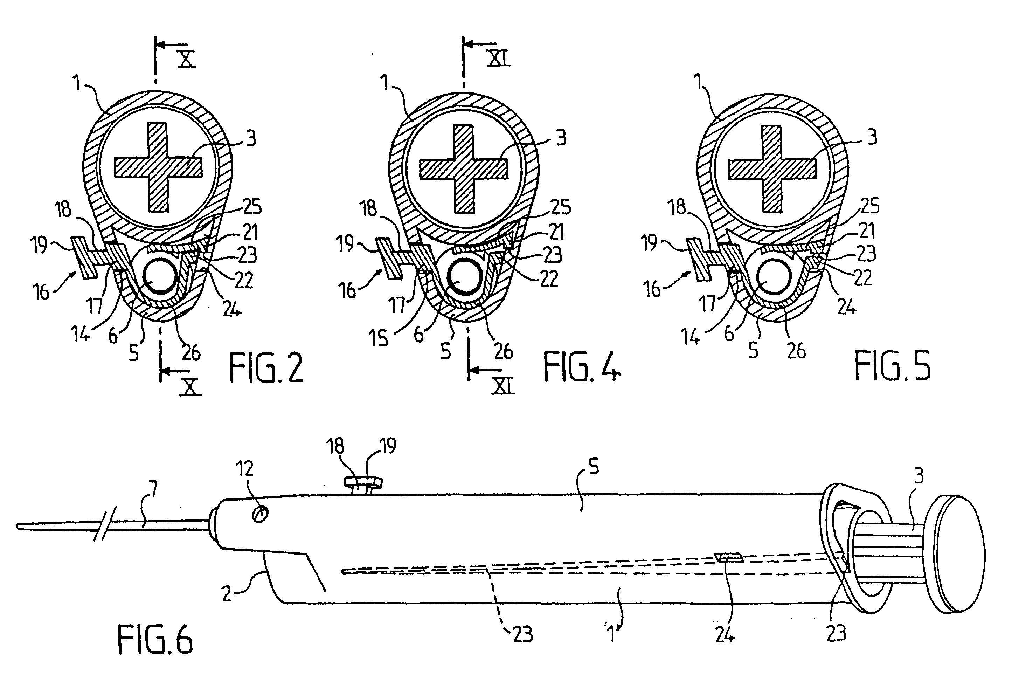

Le logement 5 a en coupe transversale une section constante définie par deux tronçons circulaires opposés et des rayons différents et par deux tronçons rectilignes reliant les tronçons circulaires. Sa forme en coupe transversale est clairement visible sur les figures 2, 4 et 5.

Le logement 5 renferme un coulisseau 6 dont l'extrémité antérieure porte une aiguille 7 comportant un conduit interne 8, cette aiguille étant emmanchée dans le coulisseau et fixée par collage.

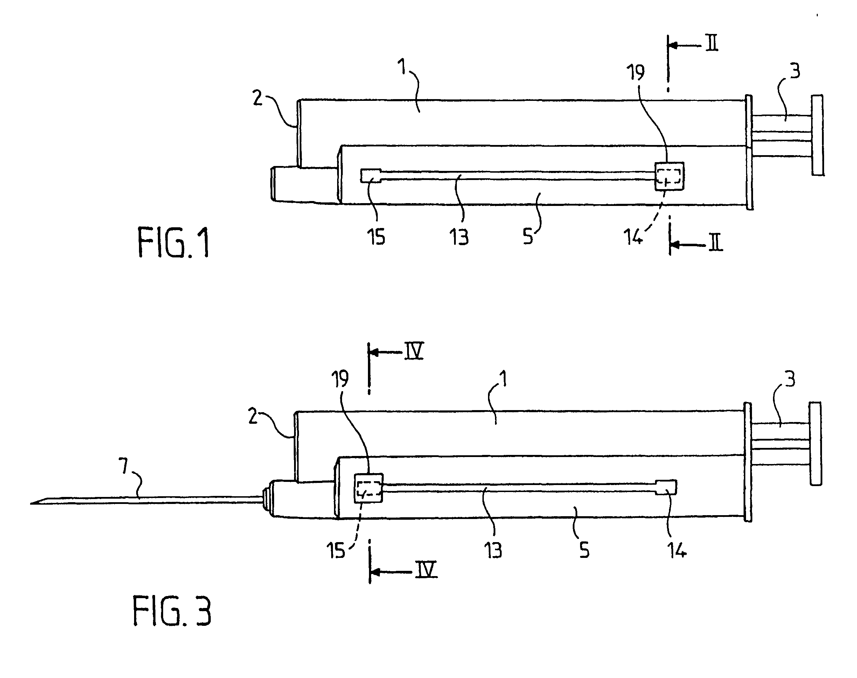

Le coulisseau 6 est déplaçable axialement dans le logement 5 entre une position extrême rétractée (représentée sur les figures 1, 7 et 10) dans laquelle l'aiguille est entièrement contenue dans le logement et une position extrême avancée (représentée sur les figures 3, 6 et 11) dans laquelle l'aiguille fait saillie hors du logement, au niveau de l'extrémité antérieure à celui-ci.

Les figures 10 et 11 montrent que l'extrémité antérieure du logement 5 est pourvue d'un alésage conique 9 dont les dimensions correspondent à celles de l'extrémité antérieure du coulisseau afin que ce dernier l'obture de manière étanche lorsqu'il est dans sa position extrême avancée.

Elles montrent également que le coulisseau comporte un conduit coudé 10 relié au conduit interne 8 de l'aiguille.

Lorsque le coulisseau est dans sa position extrême avancée, le conduit coudé 10 est en face d'un trou 11 ménagé dans la partie de la paroi latérale du corps tubulaire 1 qui est commune avec le logement 5.

Dans cette position du coulisseau, l'intérieur du corps tubulaire 1 est donc relié au conduit interne 8 de l'aiguille.

On notera ici qu'un trou 12 diamétralement opposé au trou 11 est réalisé dans la paroi latérale du logement 5 pour permettre la réalisation du trou 11.

En se référant maintenant aux figures 1 et 3, on remarquera que la paroi latérale du logement est pourvue d'une fente longitudinale 13 se terminant par des élargissements postérieur 14 et antérieur 15, ces élargissements étant rectangulaires et ayant les mêmes dimensions.

Le coulisseau 6, qui est réalisé de préférence en matière plastique, comporte des moyens de verrouillage 16 qui, en coopérant avec les élargissements 14 et 15, sont destinés à l'immobiliser dans ses positions externes rétractée et avancée.

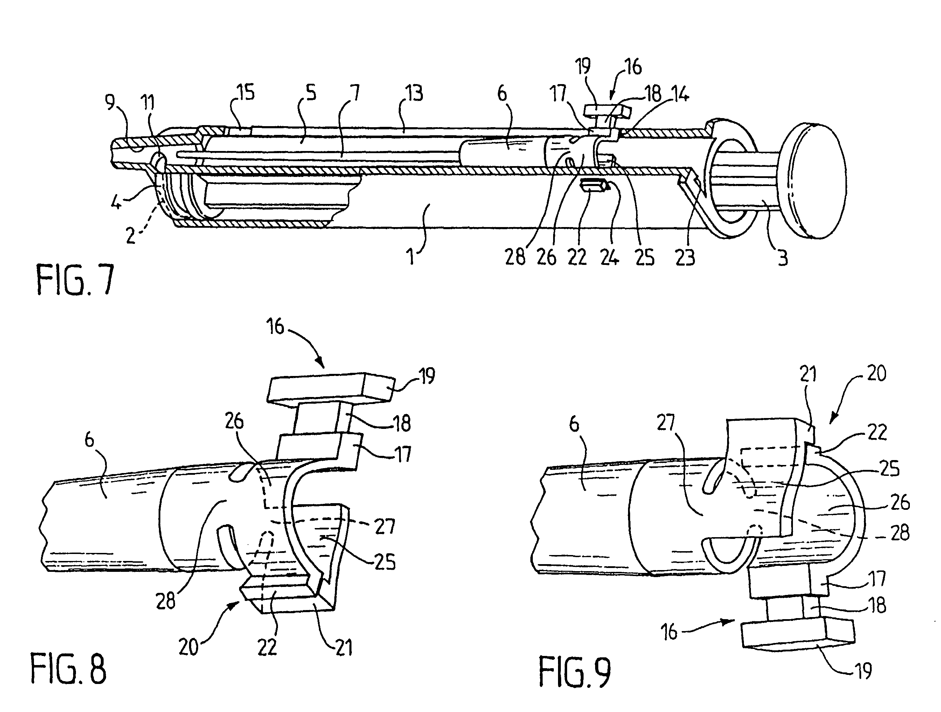

Comme le montrent clairement les figures 7 à 9, les moyens de verrouillage 16 comprennent une embase 17 dont l'étendue correspond à celle des élargissements et qui est prolongée radialement vers l'extérieur par une zone rétrécie 18 dont la largeur correspond à celle de la fente longitudinale 13, puis par un poussoir externe 19 faisant saillie lors de cette dernière.

En raison de leur position particulière sur le coulisseau 6 et compte tenu de la matière plastique utilisée pour réaliser ce dernier, les moyens de verrouillage 16 sont sollicités élastiquement radialement vers l'extérieur de sorte que pour déverrouiller le coulisseau 6, il suffit d'exercer une pression sur le poussoir 19 afin d'amener la zone rétrécie 18 au niveau de la fente longitudinale 13 et de déplacer ensuite longitudinalement le poussoir dans le sens voulu.

La seringue conforme à l'invention comprend en outre des moyens de sécurité 20 destinés à immobiliser définitivement le coulisseau 6 dans sa position extrême rétractée lorsque celui-ci est ramené dans cette position après avoir été déplacé jusque dans sa position extrême avancée.

Dans certains cas, les moyens de sécurité pourraient exercer leur action dès lors que le coulisseau 6 serait ramené dans sa position extrême rétractée après avoir été déplacé sur une distance limitée en direction de sa position extrême avancée.

En se référant en particulier aux figures 2 et 4 à 9, on constatera que les moyens de sécurité 20 comprennent un crochet 21 et un verrou 22 portés par le coulisseau 6, une rampe 23 prévue sur la face interne du logement 5, et un orifice 24 ménagé dans la paroi de ce dernier.

Lorsque le coulisseau 6 est dans sa position extrême rétractée, le crochet 21 retient le verrou 22, comme représenté sur les figures 2 et 9.

Par contre, lorsque le coulisseau est déplacé en direction de sa position extrême avancée, la rampe 23 déplace le crochet 21 en direction du corps tubulaire 1 et l'oblige ainsi à libérer le verrou 22, comme représenté sur les figures 4 et 8.

Le verrou 22 étant désolidarisé du crochet 21, il viendra automatiquement dans l'orifice 24 lorsque le coulisseau sera ramené dans sa position extrême rétractée.

Dans le mode de réalisation représenté, le crochet 21 est prévu à l'une des extrémités d'un premier élément élastique 25 prolongeant le coulisseau axialement vers l'arrière.

Le verrou 22 est quant à lui prévu à l'une des extrémités d'un second élément élastique 26 prolongeant également le coulisseau axialement vers l'arrière.

Les figures 8 et 9 montrent clairement que les éléments élastiques 25 et 26 sont disposés l'un en face de l'autre et que leurs extrémités qui portent respectivement le crochet 21 et le verrou 22 sont adjacentes.

Elles montrent également que les moyens de verrouillage 16 sont prévus à l'autre extrémité du second élément élastique 26.

Elles montrent encore que le premier élément élastique 25 est relié au coulisseau 6 par un pont 27 adjacent à son extrémité qui est opposé au crochet 21, que le second élément élastique 26 est relié au coulisseau 6 par un pont 28 approximativement équidistant de ses deux extrémités, et que les deux ponts 27 et 28 sont pratiquement diamétralement opposés.

On va maintenant décrire les opérations qu'il convient d'effectuer pour utiliser la seringue conforme à l'invention.

On précisera tout d'abord que cette seringue est commercialisée avec le coulisseau 6 dans sa position extrême rétractée, le piston 4 étant quant à lui appliqué contre le fond 2 du corps tubulaire 1, comme représenté sur la figure 10.

Dans cette situation, l'embase 17 des moyens de verrouillage s'étend dans l'élargissement postérieur 14 de la fente longitudinale 13 et s'oppose à ce que le coulisseau 6 soit déplacé en direction de l'alésage conique 9 du logement 5.

Pour déplacer le coulisseau dans cette direction, il convient d'exercer une pression sur le poussoir 19 pour amener la zone rétrécie 18 dans la fente longitudinale 13 et de pousser ensuite le poussoir 19 en direction de l'extrémité antérieure du logement 15.

Pendant le déplacement du coulisseau 6 vers sa position extrême avancée, la rampe 23 située sur la face interne du logement 5 déplace progressivement le crochet 21 en direction du corps tubulaire 1, en l'obligeant à libérer le verrou 22.

Lorsque le coulisseau 6 est dans sa position extrême avancée visible sur les figures 3, 6 et 11, l'aiguille 7 fait saillie hors du logement 5 et est prête pour l'exécution d'une injection ou d'un prélèvement, tandis que l'embase 17 fait saillie dans l'élargissement antérieur 15 de la fente longitudinale.

En outre, le verrou 22 est séparé du crochet 21, la rampe 23 ayant contraint ce dernier à se déplacer vers le corps tubulaire 1 et à libérer le verrou comme le montre la figure 4.

Le conduit coudé 10 du coulisseau 6 est quant à lui devant le trou 11 ménagé dans la partie de paroi séparant le corps 1 du logement 5, ce qui permet de relier l'intérieur du corps 1 avec le conduit interne 8 de l'aiguille 7.

On peut procéder maintenant à l'introduction du liquide à injecter en implantant l'aiguille dans le récipient contenant le liquide et en éloignant le piston 4 du fond 2.

Lorsque la quantité de liquide nécessaire est dans le corps 1, on effectue l'injection de façon habituelle.

Après l'exécution de cette opération, on ramène le coulisseau dans sa position extrême rétractée afin de rentrer l'aiguille dans le logement 5.

Cette opération est réalisée en exerçant une pression sur le poussoir 19 afin d'amener la zone rétrécie 18 dans la fente 13, et en déplaçant ce dernier vers l'élargissement postérieur 14 de la fente longitudinale 13.

Le verrou 22, qui est désolidarisé du crochet 21, frotte contre la surface interne du logement 5 pendant le déplacement du coulisseau vers sa position extrême rétractée et pénètre automatiquement dans l'orifice 24 ménagé dans la paroi du logement 5 dès que le coulisseau atteint sa position extrême rétractée.

A partir de cet instant, le coulisseau est définitivement immobilisé dans le logement, ce qui interdit toute nouvelle utilisation de la seringue.

Pour être complet, on précisera que le corps tubulaire 1 et le logement 5 sont réalisés d'une seule pièce par moulage d'une matière plastique transparente ou non.

## Revendications

1. Seringue comprenant un corps tubulaire (1) destiné à recevoir un liquide à injecter ou à prélever, un piston (4) déplaçable axialement dans le corps tubulaire, et une aiguille (7) comportant un conduit interne (8) et portée par un coulisseau (6) situé dans un logement (5) qui est parallèle et adjacent au corps tubulaire (1), le logement (5) comportant une extrémité antérieure pourvue d'un alésage conique (9) tandis que le coulisseau (6) est déplaçable axialement dans le logement entre une position extrême rétractée dans laquelle l'aiguille est entièrement contenue dans le logement et une position extrême avancée dans laquelle l'aiguille fait saillie hors du logement, et fait communiquer l'intérieur du corps tubulaire avec le conduit interne de l'aiguille lorsqu'il est dans sa position extrême avancée, **caractérisée en ce que** les dimensions de l'alésage conique (9) correspondent à celles de l'extrémité antérieure du coulisseau (6), et **en ce que** celui-ci, lorsqu'il est dans sa position extrême avancée, est situé dans l'alésage conique (9) et l'obture de manière étanche.

2. Seringue selon la revendication 1, **caractérisée en ce que** le logement (5) comporte une paroi latérale pourvue d'une fente longitudinale (13) se terminant par des élargissements postérieur (14) et antérieur (15), tandis que le coulisseau (6) comporte des moyens de verrouillage (16) destinés à coopérer avec les élargissements de la fente longitudinale pour l'immobiliser dans ses positions extrêmes.

3. Seringue selon la revendication 2, **caractérisée en ce que** les moyens de verrouillage (16) sont sollicités élastiquement radialement vers l'extérieur et comprennent une embase (17) dont l'étendue correspond à celle des élargissements (14,15) de la fente longitudinale (13) et qui se prolonge radialement vers l'extérieur par une zone rétrécie (18) dont la largeur correspond à celle de la fente longitudinale (13) puis par un poussoir externe (19) faisant saillie hors de ladite fente.

4. Seringue selon l'une quelconque des revendications précédentes, **caractérisée en ce qu'**elle comprend des moyens de sécurité (20) pour immobiliser définitivement le coulisseau (6) dans sa position extrême rétractée lorsque celui-ci est ramené dans cette position après avoir été déplacé vers ou jusqu'à sa position extrême avancée.

5. Seringue selon la revendication 4, **caractérisée en ce que** les moyens de sécurité (20) comprennent un crochet (21) et un verrou (22) portés par le coulisseau (6), le verrou (22) étant retenu par le crochet (21) lorsque le coulisseau (6) est dans sa position extrême rétractée, une rampe (23) prévue sur la face interne du logement (5) et conformée de façon à déplacer le crochet pour qu'il libère le verrou lors du déplacement du coulisseau de sa position extrême rétractée vers sa position extrême avancée, et un orifice (24) ménagé dans la paroi du logement (5) pour recevoir le verrou libéré lorsque le coulisseau (6) est ramené dans sa position extrême rétractée.

6. Seringue selon la revendication 5, **caractérisée en ce que** le crochet (21) est prévu à l'une des extrémités d'un premier élément élastique (25) prolongeant le coulisseau (6) axialement vers l'arrière tandis que le verrou (22) est prévu à l'une des extrémités d'un second élément élastique (26) prolongeant également le coulisseau axialement vers l'arrière, les premier et second éléments (25,26) étant disposés l'un en face de l'autre de telle sorte que leurs extrémités portant respectivement le crochet et le verrou soient adjacentes.

7. Seringue selon la revendication 2 ou 3 et la revendication 5, **caractérisée en ce que** les moyens de verrouillage (16) sont prévus à l'autre extrémité du second élément (26) prolongeant le coulisseau (6) axialement vers l'arrière.

8. Seringue selon la revendication 6 ou 7, **caractérisée en ce que** le premier élément (25) est relié au coulisseau (6) par un pont (27) adjacent à son extrémité qui est opposée au crochet (21) tandis que le second élément (26) est relié au coulisseau (6) par un pont (28) approximativement équidistant de ses deux extrémités.

9. Seringue selon la revendication 8, **caractérisée en ce que** les deux ponts (27,28) sont pratiquement diamétralement opposés.

## Patentansprüche

1. Spritze, welche einen rohrförmigen Korpus (1) aufweist, der zur Aufnahme einer zu injizierenden oder abzunehmenden Flüssigkeit bestimmt ist, femer einen Kolben (4), der in dem rohrförmigen Korpus in axialer Richtung verschieblich ist, und eine Nadel (7) mit einem innen liegenden Kanal (8), welche ein Schiebers (6) trägt, der sich in einer Aufnahme (5) befindet, die parallel zum rohrförmigen Korpus (1) verläuft und sich nahe diesem befindet, wobei die Aufnahme (5) ein vorderes Ende besitzt, das mit einer konischen Ausbohrung (9) versehen ist, wohingegen der Schieber (6) in axialer Richtung in der Aufnahme zwischen einer am weitesten zurückgezogenen Position, in welcher die Nadel vollständig in der Aufnahme aufgenommen ist, und einer am weitesten vorgeschobenen Position verschieblich ist, in welcher die Nadel aus der Aufnahme vorsteht und eine Strömungsverbindung zwischen dem Innenraum des rohrförmigen Korpus und dem innen liegenden Kanal der Nadel herstellt, wenn diese sich in ihrer am weitesten vorgeschobenen Position befindet, **dadurch gekennzeichnet, dass** die Abmessungen der konischen Ausbohrung (9) den Abmessungen des vorderen Endes des Schiebers (6) entsprechen, und dass der Schieber in der konischen Ausbohrung (9) liegt und diese unter Abdichtung verschließt, wenn er sich in seiner am weitesten vorgeschobenen Stellung befindet.

2. Spritze nach Anspruch 1, **dadurch gekennzeichnet, dass** die Aufnahme (5) eine Seitenwandung besitzt, die mit einem in Längsrichtung verlaufenden Schlitz (13) versehen ist, welcher in hinten (14) und vorne (15) liegende Erweiterungen ausläuft, wohingegen der Schieber (6) Verriegelungsmittel (16) aufweist, die zum Zusammenwirken mit den Erweiterungen des in Längsrichtung verlaufenden Schlitzes zur Feststellung des Schiebers in seinen Endpositionen bestimmt sind.

3. Spritze nach Anspruch 2, **dadurch gekennzeichnet, dass** die Verriegelungsmittel (16) in radialer Richtung elastisch nach außen gespannt sind und einen Ansatz (17) aufweisen, dessen Erstreckung der Erstreckung der Erweiterungen (14, 15) des in Längsrichtung verlaufenden Schlitzes (13) entspricht und der sich in radialer Richtung über einen enger gestalteten Bereich (18), dessen Breite der Breite des in Längsrichtung verlaufenden Schlitzes (13) entspricht, und über einen außen liegenden Drücker (19) nach außen fortsetzt, welcher aus dem Schlitz vorsteht.

4. Spritze nach einem der vorhergehenden Ansprüche, **dadurch gekennzeichnet, dass** sie Sicherheitseinrichtungen (20) zum absoluten Feststellen des Schiebers (6) in seiner am weitesten zurückgezogenen Position aufweist, wenn dieser in diese Position zurückgeführt wurde, nachdem er in seine vorgeschobene Position bzw. bis zu dieser verschoben wurde.

5. Spritze nach Anspruch 4, **dadurch gekennzeichnet, dass** die Sicherheitseinrichtungen (20) ein Hakenteil (21) und ein Riegelteil (22) aufweisen, welche der Schieber (6) trägt, wobei das Riegelteil (22) mittels des Hakenteils (21) gehalten ist, wenn sich der Schieber (6) in seiner am weitesten zurückgezogenen Position befindet, und ferner eine Schrägfläche (23), die auf der innen liegenden Fläche der Aufnahme (5) vorgesehen und in der Weise an die Form angepasst ist, dass sie das Hakenteil so verschiebt, dass dieses das Riegelteil während der Verlagerung des Schiebers aus seiner am weitesten zurückgezogenen Position zu seiner am weitesten vorgeschobenen Position freigibt, und eine Öffnung (24), die in der Wandung (5) der Aufnahme ausgebildet ist, um das freigegebene Riegelteil aufzunehmen, wenn der Schieber (6) in seine am weitesten zurückgezogene Position zurückgeführt ist.

6. Spritze nach Anspruch 5, **dadurch gekennzeichnet, dass** das Hakenteil (21) an einem der Enden eines ersten elastischen Elements (25) vorgesehen ist, welches den Schieber (6) in axialer Richtung nach hinten verlängert, wohingegen das Riegelteil (22) an einem der Enden eines zweiten elastischen Elements (26) vorgesehen ist, welches ebenfalls den Schieber in axialer Richtung nach hinten verlängert, wobei das erste und das zweite Element (25, 26) einander gegenüber in der Weise angeordnet sind, dass ihre Enden, die jeweils das Hakenteil bzw. das Riegelteil tragen, einander benachbart liegen.

7. Spritze nach Anspruch 2 oder 3 und Anspruch 5, **dadurch gekennzeichnet, dass** die Verriegelungseinrichtungen (16) am anderen Ende des zweiten Elements (26) vorgesehen sind, welche den Schieber (6) in axialer Richtung nach hinten verlängern.

8. Spritze nach Anspruch 6 oder 7, **dadurch gekennzeichnet, dass** das erste Element (25) mit dem Schieber (6) über Brückenteil (27) verbunden ist, wobei das Brückenteil dessen Ende benachbart ist, das dem Hakenteil (21) gegenüber liegt, wohingegen das zweite Element (26) mit dem Schieber (6) über ein Brückenteil (28) verbunden ist, das sich in etwa in gleichem Abstand von dessen beiden Enden befindet.

9. Spritze nach Anspruch 8, **dadurch gekennzeichnet, dass** sich die beiden Brückenteile (28, 28) praktisch diametral einander gegenüber liegen.

## Claims

1. A syringe comprising a tubular body (1) for receiving a liquid to be injected or sampled, a piston (4) axially movable within the tubular body, and a needle (7) provided with an inner conduit (8) and carried by a slider (6) located in a housing (5), which is parallel with and adjacent to the tubular body (1), said housing (5) having a front end provided with a cone-shaped bore (9), while the slider (6) is axially movable within the housing between a retracted extreme position, in which the needle is entirely contained in the housing, and an advanced extreme position, in which the needle is projecting out of the housing, and causes the inside of the tubular body to communicate with the inner conduit of the needle, when in its advanced extreme position, **characterized in that** the dimensions of the cone-shaped bore (9) correspond to those of the front end of the slider (6), and **in that** said slider, when in its advanced extreme position, is located in the cone-shaped bore (9) and tightly seals said bore.

2. The syringe according to claim 1, **characterized in that** the housing (5) comprises a side wall with a longitudinal slot (13), that terminates in rear (14) and front (15) widened portions, while the slider (6) comprises locking means (16) provided for cooperating with the widened portions of the slot in order to immobilize it in its extreme positions.

3. The syringe according to claim 2, **characterized in that** the locking means (16) are resiliently biased radially outwards and comprise a base (17), the extent of which corresponds to that of the widened portions (14, 15) of the longitudinal slot (13) and which is continued radially outwards by a narrowed region (18), the width of which corresponds to that of the longitudinal slot (13), and further by an outer push member (19) projecting out of said slot.

4. The syringe according to any of the preceding claims, **characterized in that** it comprises safety means (20) for definitely immobilizing the slider (6) in its retracted extreme position, when returned to said position after being moved towards or up to its advanced extreme position.

5. The syringe according to claim 4, **characterized in that** the safety means (20) comprise a hook (21) and a latch (22) carried by the slider (6), said latch (22) being retained by the hook (21) when the slider (6) is in its retracted extreme position, a ramp (23) provided on the inner face of the housing (5) and so configured as to move the hook for disengaging the latch when the slider is moved from its retracted extreme position towards its advanced extreme position, and an aperture (24) formed in the wall of the housing (5) for receiving the disengaged latch when the slider (6) is returned to its retracted extreme position.

6. The syringe according to claim 5, **characterized in that** the hook (21) is provided at one end of a first resilient member (25) extending the slider (6) axially backwards, while the latch (22) is provided at one end of a second resilient member (26) also extending the slider axially backwards, said first and second members (25, 26) being disposed one in front of the other so that their ends carrying the hook and the latch, respectively, are adjacent.

7. The syringe according to claim 2 or 3 and claim 5, **characterized in that** the locking means (16) are provided at the other end of the second member (26) extending the slider (6) axially backwards.

8. The syringe according to claim 6 or 7, **characterized in that** the first member (25) is connected to the slider (6) through a bridge (27) adjacent its end opposed to the hook (21), while the second member (26) is connected to the slider (6) through a bridge (28) approximately equally spaced from its two ends.

9. The syringe according to claim 8, **characterized in that** the two bridges (27, 28) are substantially diametrically opposed.
